# EUROPÄISCHE PATENTSCHRIFT

(11) **EP 4 499 204 B1**
(45) Veröffentlichungstag und Bekanntmachung des Hinweises auf die Patenterteilung: **18.06.2025**
(21) Anmeldenummer: 23706872.1
(22) Anmeldetag: 22.02.2023
(51) Int. Cl.: A61N 1/36, A61N 1/04

(54) **VORRICHTUNG ZUR AURIKULÄREN PUNKTUALSTIMULATION**
DEVICE FOR AURICULAR PUNCTURE STIMULATION
DISPOSITIF DE STIMULATION PONCTUELLE AURICULAIRE

(30) Priorität: 29.03.2022 EP 22020137
(43) Veröffentlichungstag der Anmeldung: 05.02.2025
(73) Patentinhaber: Aurimod GmbH, 1030 Wien (AT)
(72) Erfinder: KANIUSAS, Eugenijus, 1180 Wien (AT); KAMPUSCH, Stefan, 1020 Wien (AT); THÜRK, Florian, 1210 Wien (AT)
(74) Vertreter: SONN Patentanwälte GmbH & Co KG
(86) Internationale Anmeldenummer: PCT/IB2023/051610
(87) Internationale Veröffentlichungsnummer: WO 2023/187492

(56) Entgegenhaltungen:
- WO-A1-2022/018289
- AU-A1- 2020 313 858
- US-A1- 2012 016 431
- US-A1- 2017 113 042
- US-A1- 2021 259 879

## Beschreibung

Die Erfindung betrifft eine Vorrichtung zur aurikulären Punktualstimulation eines Patienten, umfassend einen Stromgenerator zur Erzeugung von Stimulationsstromimpulsen und vom Stromgenerator ausgehende elektrische Leitungen zur Verbindung mit je einer am Ohr zu positionierenden Elektrode, von denen wenigstens zwei mit den Stimulationsstromimpulsen beaufschlagbar sind, und weiters umfassend Messmittel zur Ermittlung wenigstens eines physiologischen Messwerts des Patienten.

Verfügbare Stimulationsgeräte für die elektrische Stimulation z.B. des aurikulären Vagusnervs werden in der Therapie verschiedenster Erkrankungen, wie z.B. chronischer und akuter Schmerzen, Epilepsie und Depression, eingesetzt. Bei aurikulären Punktualstimulationsgeräten, wie sie z.B. aus der WO 2011/030210 A1 bekannt geworden sind, werden elektrische Stimulationspulse über Nadelelektroden eingeleitet, die an vorgegebenen Stellen der Ohrmuschel in die Haut eingestochen werden und dort für den Behandlungszeitraum von z.B. mehreren Tagen verbleiben.

Die WO 2022/018289 A1 beschreibt ein Verfahren und eine entsprechende Vorrichtung, mit der Stimulationsimpulse zur aurikulären Punktualstimulation abgegeben werden können und bei der zusätzlich eine Impedanzmessung erfolgt. Eine Impedanz-Plethysmographie ist jedoch nicht Gegenstand dieser Veröffentlichung.

Die Stimulation ermöglicht zum Beispiel die vorteilhafte Beeinflussung von Schmerzverarbeitung und Schmerzwahrnehmung. Auch wird durch die Stimulation die sympathovagale Balance im autonomen Nervensystem positiv beeinflusst. In der Folge verändert sich der aktuelle physiologische Zustand des Patienten dynamisch, gemessen als Veränderung der Hirnaktivität, der Herzrate, der Atemrate, des Blutdrucks, der lokalen Durchblutung und anderer Parameter.

Die Stimulationsparameter sind bei aktuellen technischen Lösungen entweder fix eingestellt oder durch den Arzt und/oder Patienten bei der Applikation bzw. Nachjustierung des Gerätes einstellbar. Die Parameter berücksichtigen somit nicht oder nur sehr schlecht den momentanen und über den Therapieverlauf eventuell stark veränderlichen physiologischen Zustand des Patienten. Fehlendes physiologisches Feedback bedingt eine Über- oder Unterstimulation des Patienten über den gesamten Therapiezeitraum, wodurch kritische Therapieziele verfehlt werden. So können zum Beispiel Amplitude, Frequenz, Ein- und Ausschaltzeiten nicht optimal kontrolliert werden. Bei einer Überstimulation kann es zu zusätzlichen Schmerzen kommen, mit dem weiteren Nachteil, dass die Batterie des Gerätes schneller entladen wird. Die Unterstimulation lässt individuelle Nöte des Patienten außer Acht und verfehlt somit die kritischen Therapieziele. So wird der Nerv zum Beispiel nicht ausreichend gereizt, um die gewünschte therapeutische Wirkung zu erreichen.

Derzeit verfügbare Konzepte zu anpassungsfähigen Stimulationsgeräten verwenden zusätzlich implantierte oder interne und externe Sensoren, um ein physiologisches Feedbacksignal zur Individualisierung der Therapie zu realisieren. Kardiovaskuläre und kardiorespiratorische Parameter können zur gezielten Steuerung oder sogar zur rückgekoppelten Regelung der augenblicklichen Stimulationsparameter verwendet werden, um eine erkrankungs- und patientenspezifische Therapie mittels feedbackbasierter Stimulation zu ermöglichen.

Ein wesentlicher Nachteil bestehender Konzepte der Stimulationsgeräte ist die Notwendigkeit mindestens eines internen oder externen Sensors (z.B. PPG Sensor, etc.), der ein physiologisches Sensorsignal zur individualisierten Regelung der Stimulation liefert. Speziell externe Sensoren behindern nicht nur den Patienten selbst, sondern stellen auch einen wesentlichen Zeitaufwand und Kostenfaktor bei der Einschulung, und bei Beginn und Durchführung der Therapie dar. Die Möglichkeiten interner Sensoren sind oft begrenzt in Bezug auf die Möglichkeit aussagekräftige Daten zu erfassen, bzw. brauchen diese auch zusätzlich Platz und Energie.

Die vorliegende Erfindung zielt daher darauf ab, eine Vorrichtung zur aurikulären Punktualstimulation z.B. des Vagusnervs dahingehend zu verbessern, dass die Ermittlung eines physiologischen Messwerts vereinfacht wird, wobei insbesondere die oben angesprochenen Nachteile vermieden werden sollen.

Zur Lösung dieser Aufgabe besteht die Erfindung bei einer Vorrichtung der eingangs genannten Art im Wesentlichen darin, dass die Messmittel zur Ermittlung des wenigstens einen physiologischen Messwerts mittels Impedanz-Plethysmographie über die Elektroden ausgebildet sind. Dadurch, dass der physiologische Messwert unter Verwendung des Prinzips der Impedanz-Plethysmographie erfolgt, können die für die Stimulation z.B. des Vagusnerv ohnehin vorhandenen Elektroden für die Messung der Gewebeimpedanz im Ohr mitverwendet werden. Es wurde somit eine Sensorimplementierung gefunden, die keine zusätzlichen Sensoren benötigt, die zu implantieren wären oder die an einer Messstelle am Körper angebracht werden müssten. Dadurch wird eine Beeinträchtigung des Patienten durch zusätzliche Sensoren vermieden und damit zusätzlich die Patienten-Compliance verbessert. Weiters werden der Zeitaufwand und die Kosten bei der Einschulung sowie bei Beginn und Durchführung der Therapie verringert. Die Erfindung ist jedoch nicht darauf beschränkt, dass die Stimulation und die Impedanzmessung über dieselben Elektroden erfolgt. Vielmehr können für die Impedanzmessung andere Elektroden verwendet werden als für die Stimulation. Bevorzugt wird jedoch wenigstens eine Elektrode, über welche die Stimulation erfolgt, für die Impedanzmessung mitverwendet.

Die Impedanz-Plethysmographie ist ein Verfahren, mit dem der elektrische Wechselstromwiderstand, d.h. die Impedanz, eines Körperabschnittes erfasst werden kann. Da Blut im Vergleich zu anderen Gewebearten ein guter elektrischer Leiter ist, führen Änderungen des Blutvolumens an der Messstelle zu messbaren Impedanzänderungen. Zur Impedanzmessung wird über zwei Elektroden ein hochfrequenter Wechselstrom in das Ohr eingeprägt, der unterschwellig ist und die aurikulären Nerven daher nicht reizt. Die Messung der Impedanz erfolgt in der Regel über das Abgreifen der Spannung zwischen zwei Elektroden, die sich in Abhängigkeit von der Durchblutung verändert. Folglich kann auf diese Weise die Durchblutung erfasst und analysiert werden.

Erfindungsgemäß ist in diesem Zusammenhang vorgesehen, dass der Stromgenerator zur Erzeugung eines Wechselstroms ausgebildet ist, der über zwei der elektrischen Leitungen und die zugehörigen Elektroden in das Ohr einleitbar ist, und dass die Messmittel eine Messschaltung zur Messung einer zwischen zwei Elektroden abgegriffenen Gewebeimpedanz aufweisen.

Die Impedanzmessung kann hierbei unter Verwendung von zwei, drei oder vier Elektroden oder mehr erfolgen. Im Fall von drei Elektroden, umfassend zwei äußere und eine zwischen diesen angeordnete mittlere Elektrode, erfolgt die Einbringung des Wechselstroms vorteilhaft über die äußeren Elektroden und der Spannungsabgriff erfolgt über die mittlere und eine der beiden äußeren Elektroden. Im Fall von vier Elektroden, umfassend zwei äußere und zwei entlang des Strompfades zwischen diesen angeordnete mittlere Elektroden, erfolgt die Einbringung des Wechselstroms vorteilhaft über die äußeren Elektroden und der Spannungsabgriff erfolgt über die beiden mittleren Elektroden.

Die Impedanzmessung kann derart erfolgen, dass ein Wechselstrom mit einer vorgegebenen Stromstärke eingebracht wird und der Spannungsabfall an den Messelektroden ermittelt wird. Alternativ kann Wechselstrom mit einer vorgegebenen Spannung eingebracht und die sich über die Messelektroden ergebende Stromstärke ermittelt werden.

Die lokale Gewebeimpedanz und ihre dynamischen Veränderungen reflektieren lokale Perfusionsverhältnisse im Ohr und erlauben damit einen individuellen und zeitvarianten Aufschluss über die kardiale und respiratorische Situation des Patienten.

Der wenigstens eine physiologische Messwert, der mit der erfindungsgemäßen Vorrichtung ermittelt wird, kann ein primärer Messwert oder ein abgeleiteter, sekundärer Messwert sein. Bei dem primären Messwert handelt es sich um die Gewebeimpedanz, die proportional zu der über die Elektroden abgegriffenen Spannung ist und von der Messschaltung erfasst werden kann. Aus der Gewebeimpedanz oder aus dem zeitlichen Verlauf der Gewebeimpedanz können abgeleitete Messwerte ermittelt werden. Dies gelingt beispielsweise durch Anwendung von Signalverarbeitungsverfahren, umfassend, Maximal- und Minimalwertdetektion, Frequenzfilter, Fourier-Analyse und Wavelet-Transformationen. Beispiele für abgeleitete Messwerte umfassen die Herzrate, die Herzratenvariabilität, die Durchblutung, die Gefäßsteifigkeit und die Atemfrequenz. Die Signalverarbeitung kann hierbei in der erfindungsgemäßen Vorrichtung erfolgen oder in einer externen Vorrichtung, der die primären Messwerte zur Verfügung gestellt werden.

Im ersteren Fall sieht eine bevorzugte Weiterbildung der Erfindung vor, dass die Messmittel eine mit der Messschaltung verbundene Signalverarbeitungsschaltung aufweisen, die ausgebildet ist, um aus dem zeitlichen Verlauf der Gewebeimpedanz den wenigstens einen physiologischen Messwert, wie z.B. die Herzrate, die Herzratenvariabilität, die Durchblutung, die Gefäßsteifigkeit und/oder die Atemfrequenz, zu ermitteln. Hierbei handelt es sich um Messwerte, welche Rückschlüsse auf den Zustand des vegetativen Nervensystems des Patienten sowie auf die Wirkung der Stimulationstherapie erlauben. Die Herzratenvariabilität beispielsweise ist ein Surrogatparameter für das sympathovagale Gleichgewicht bzw. den Zustand des autonomen Nervensystems des Patienten.

Der wenigstens eine physiologische Messwert und seine zeitliche Änderung können dazu verwendet werden, den therapeutischen Erfolg der Punktualstimulation z.B. des Vagusnerv zu beobachten und aufzuzeichnen, beispielsweise über mehrere Wochen. Die Aufzeichnung des wenigstens einen physiologischen Messwerts und von dessen zeitlicher Änderung kann bloß Dokumentationszwecken dienen. Bevorzugt ist jedoch vorgesehen, dass der wenigstens eine physiologische Messwert oder dessen zeitliche Änderung zur Regelung der Stimulationstherapie herangezogen wird, z.B. im Sinne erhöhter Stimulationsfrequenz- und/oder Amplitude bei schlechtem Erfolg oder Verringerung dieser Parameter bei gutem Erfolg. Die Messwerte können beispielsweise - im allereinfachsten Fall - als adaptive Schwellwerte für die individuelle Erhöhung oder Reduktion der Stimulationsstärke oder der Stimulationsfrequenz verwendet werden. Beispielsweise erhöhen starke Schmerzen die momentane Herzrate, welche dann aufgrund des physiologischen Feedbacks zu einer verstärkten Nervenstimulation und somit zu einer verstärkten Schmerzlinderung führen könnte.

Eine bevorzugte Weiterbildung der erfindungsgemäßen Vorrichtung sieht zu diesem Zweck vor, dass eine Steuerschaltung vorgesehen ist, die mit dem Stromgenerator zur Änderung wenigstens eines Stimulationsstromparameters, wie z.B. der Pulsfrequenz, der Länge eines Bursts von Stimulationspulsen, der Stromamplitude und/oder des Tastverhältnisses der Stimulationsstromimpulse, zusammenwirkt. Besonders bevorzugt wirken die Messmittel hierbei mit der Steuerschaltung zusammen, um den wenigstens einen Stimulationsstromparameter in Abhängigkeit von dem wenigstens einen physiologischen Messwert einzustellen.

Die Stimulationsstromparameter können beispielsweise derart verstellt werden, dass bei einer Erhöhung der Herzrate oder einer Verringerung der Herzratenvariabilität die Stimulationsfrequenz oder Burstlänge erhöht wird, oder das das Tastverhältnis erhöht wird, d.h. die Einschaltdauer erhöht und/oder die Ausschaltdauer verringert wird. Auch kann die Stimulation durch bestimmte Ereignisse getriggert werden, z.B. kann eine Stimulation im Rhythmus der Herzrate oder nur während der Ausatmung erfolgen.

Die der Nervenstimulation dienende Stimulationsstromimpulsfolge und der für die Messung der Gewebeimpedanz angewendete Wechselstrom müssen sich nicht notwendigerweise voneinander unterscheiden. Der Stimulationsstrom kann somit auch zur Messung der Gewebeimpedanz herangezogen werden, wenn er als Wechselstrom ausgebildet ist.

Bevorzugt können die der Nervstimulation dienende Stimulationsstromimpulsfolge und der für die Messung der Gewebeimpedanz angewendete Wechselstrom sich jedoch im Hinblick auf die unterschiedliche zu erzielende Wirkung zumindest hinsichtlich ihrer Stromamplitude und ihrer Frequenz voneinander unterscheiden. Für die Impedanzmessung ist eine wesentlich höhere Frequenz und eine geringere Stromamplitude vorteilhaft als für die Nervenstimulation. Der Stromgenerator ist daher zur Erzeugung unterschiedlicher Stromamplituden und Frequenzen geeignet.

Bevorzugt ist hierbei vorgesehen, dass der Stromgenerator zur Erzeugung des Wechselstroms mit einer Wechselstromfrequenz von 5-100kHz ausgebildet ist. Demgegenüber ist der Stromgenerator bevorzugt zur Erzeugung der Stimulationsstromimpulse mit einer Pulsfrequenz von < 1kHz ausgebildet.

Hinsichtlich der Stromamplitude ist bevorzugt vorgesehen, dass der Stromgenerator zur Erzeugung des Wechselstroms mit einer Stromamplitude von < 2mA ausgebildet ist. Ein derartig geringer Strom liegt je nach Frequenz und Elektrodenausformung unterhalb der Stimulationsschwelle z.B. des Vagusnervs. Für die Zwecke der Stimulation ist der Stromgenerator bevorzugt zur Erzeugung der Stimulationsstromimpulse mit einer Stromamplitude von > 5mA ausgebildet. Die Stimulationsstromimpulse können hierbei bevorzugt eine alterierende Polarität aufweisen.

Da die Stimulationsschwelle jedoch von zahlreichen Faktoren abhängt, sind absolute Grenzwerte der Stromamplitude nicht immer zutreffend. Gemäß einer alternativen Ausbildung ist daher vorgesehen, dass der Stromgenerator zur Erzeugung der Stimulationsstromimpulse mit einer Stromamplitude ausgebildet ist, die mindestens dem 2-fachen, vorzugsweise mindestens dem 3-fachen, der Stromamplitude des Wechselstroms beträgt.

Die Stimulationsstromimpulse und der für die Impedanzmessung angewendete Wechselstrom können sich auch hinsichtlich der Puls- bzw. Wellenform unterscheiden. Der Wechselstrom zur Messung kann bevorzugt sinusförmig ausgebildet sein. Die Stimulationsstromimpulse hingegen können im Sinne einer Rechteckschwingung rechteckförmig ausgebildet sein.

Die Impedanzmessung und die Nervstimulation können abwechselnd vorgenommen werden, um eine gegenseitige Beeinflussung der beiden Vorgänge zu vermeiden. Die Erfindung sieht hierbei vor, dass der Stromgenerator zur Erzeugung mehrerer Sequenzen von Stimulationsstromimpulsen mit Pausen zwischen den Sequenzen und zur Erzeugung des Wechselstroms in zumindest einer der Pausen ausgebildet ist.

Alternativ kann während der Nervstimulation ein zusätzlicher unterschwelliger Wechselstrom appliziert werden, sodass die Stimulationsstromimpulse mit dem der Impedanzmessung dienenden Wechselstrom überlagert werden. Dabei wird die resultierende zusätzliche Überspannung gemessen, um aus dem Verhältnis der beiden die Gewebeimpedanz zu errechnen.

Wie bereits erwähnt, kann der wenigstens eine physiologische Messwert, d.h. die Gewebeimpedanz oder ein daraus abgeleiteter Wert, wie z.B. die Herzrate, die Herzratenvariabilität, die Gefäßfüllung, die Atemfrequenz und dgl., in der Vorrichtung selbst oder in einer externen Vorrichtung ausgewertet und ggf. als Regelgröße für die Einstellung der Nervstimulationsparameter herangezogen werden. Für die externe Auswertung ist die erfindungsgemäße Vorrichtung bevorzugt derart ausgebildet, dass die Vorrichtung eine Kommunikationsschnittstelle aufweist, welcher der wenigstens eine physiologische Messwert oder dessen zeitliche Änderung zugeführt ist und die zur Übermittlung des wenigstens einen physiologischen Messwerts oder von dessen zeitlicher Änderung an ein externe Empfangseinrichtung ausgebildet ist.

Um hierbei eine bidirektionale Kommunikation zu ermöglichen, ist die Kommunikationsschnittstelle bevorzugt zum Empfangen von Steuerbefehlen ausgebildet, die der Steuerschaltung zuführbar sind, um die Stimulationsstromparameter in Abhängigkeit von den Steuerbefehlen einzustellen.

Die Erfindung wird nachfolgend anhand von in der Zeichnung schematisch dargestellten Ausführungsbeispielen näher erläutert. In dieser zeigen Fig. 1 eine schematische Darstellung der erfindungsgemäßen Vorrichtung, Fig. 2 die Anordnung von Nadelelektroden an einem menschlichen Ohr mit einer Darstellung von Spannung und Strom für die Vagusnervstimulation, Fig. 3 die Anordnung von Nadelelektroden an einem menschlichen Ohr mit einer Darstellung von Spannung und Strom für die Gewebeimpedanzmessung, Fig. 4 eine Abfolge von Stimulationsstromimpulsen und des Wechselstroms und Fig. 5 das aus der Gewebeimpedanzmessung erhaltene Signal.

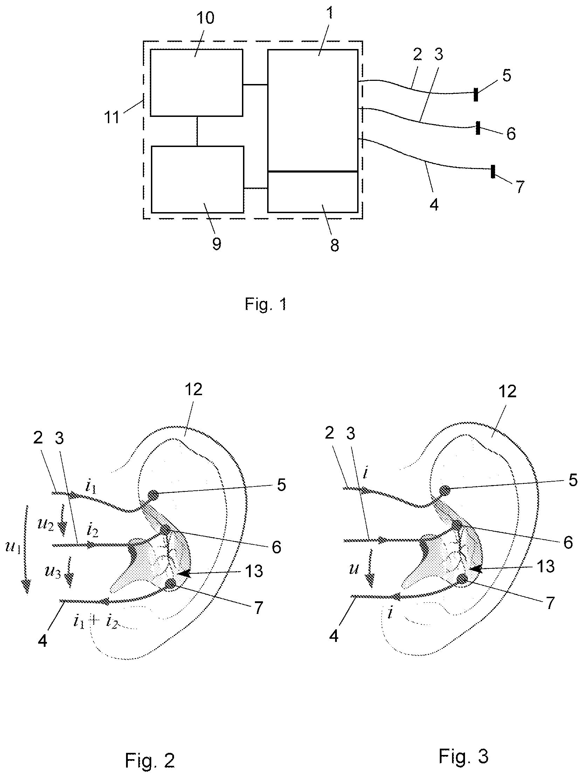

Fig. 1 zeigt eine beispielhafte Ausführung der erfindungsgemäßen Vorrichtung umfassend einen von einer nicht dargestellten Batterie gespeisten Stromgenerator 1, an den elektrische Leitungen 2, 3 und 4 angeschlossen sind, an deren Enden je eine am Ohr zu positionierende Nadelelektrode 5, 6 bzw. 7 angeordnet ist. Der Stromgenerator 1 ist zur Erzeugung von Stimulationsstromimpulsen ausgebildet, die über die Leitungen 2, 3, 4 und die zugehörigen Elektroden 5, 6, 7 in das Ohr eingebracht werden. Weiters ist der Stromgenerator 1 zur Erzeugung eines Wechselstroms ausgebildet, der z.B. über die elektrischen Leitungen 2 und 4 sowie die entsprechenden Elektroden 5 und 7 in das Ohr einleitbar ist. Zur Messung der z.B. über die Elektroden 6 und 7 abgegriffenen Gewebeimpedanz ist eine Messschaltung 8 vorgesehen.

Mit der Messschaltung 8 ist eine Signalverarbeitungsschaltung 9 verbunden, der die Messwerte der Messschaltung 8 zugeführt sind und die ausgebildet ist, um aus dem zeitlichen Verlauf der Gewebeimpedanz wenigstens einen physiologischen Messwert, wie z.B. die Herzrate, die Herzratenvariabilität, die Durchblutung, die Gefäßsteifigkeit und/oder die Atemfrequenz, zu ermitteln. Weiters ist eine Steuerschaltung 10 vorgesehen, die mit dem Stromgenerator 1 zur Änderung wenigstens eines Stimulationsstromparameters, wie **z.B.** der Pulsfrequenz und/oder der Stromamplitude der Stimulationsstromimpulse, zusammenwirkt. Die Veränderung des wenigstens einen Stimulationsstromparameters kann hierbei in Abhängigkeit der Gewebeimpedanz oder des von der Signalverarbeitungsschaltung 9 ermittelten physiologischen Messwerts bzw. von dessen zeitlichen Verlauf erfolgen, zu welchem Zweck der Messwert der Steuerschaltung 10 von der Signalverarbeitungsschaltung 9 zugeführt wird.

Der Stromgenerator 1, die Messschaltung 8, die Signalverarbeitungsschaltung 9 und die Steuerschaltung 10 sind in einem Gehäuse 11 angeordnet, das in der Nähe des Ohrs, z.B. hinter dem Ohr, angebracht werden kann. Alternativ ist eine Anbringung auch an einer anderen Körperstelle, wie z.B. an der Brust, denkbar. Der Stromgenerator 1, die Messschaltung 8, die Signalverarbeitungsschaltung 9 und die Steuerschaltung 10 können als gesonderte Baueinheiten ausgebildet oder in einer gemeinsamen elektronischen Schaltung implementiert sein.

Fig. 2 zeigt das menschliche Ohr 12 mit Blutgefäßen und den afferenten Vagusnerv-Ästen 13. Im Bereich der Vagusnerv-Äste sind die Nadelelektroden 5, 6 und 7 in das Gewebe eingestochen, wobei über die Leitung 2 eine Folge von Stimulationsimpulsen mit dem Strom *i₁* und über die Leitung 3 eine Folge von Stimulationsimpulsen mit dem Strom *i₂* eingebracht werden und der Stromrückfluss *i₁*+*i₂* über die Leitung 4 erfolgt. Es ergibt sich zwischen den Leitungen 2 und 4 eine Spannung *u₁,* zwischen den Leitungen 2 und 3 eine Spannung *u₂* und zwischen den Leitungen 3 und 4 eine Spannung *u₃.*

Fig. 3 zeigt die Messung der Gewebeimpedanz im Ohr mit Hilfe eines Wechselstroms. Dabei wird ein Wechselstromkreis über die Leitungen 2 und 4 erzeugt und die Spannung u wird über die Leitungen 3 und 4 abgegriffen und in der Messschaltung 8 gemessen.

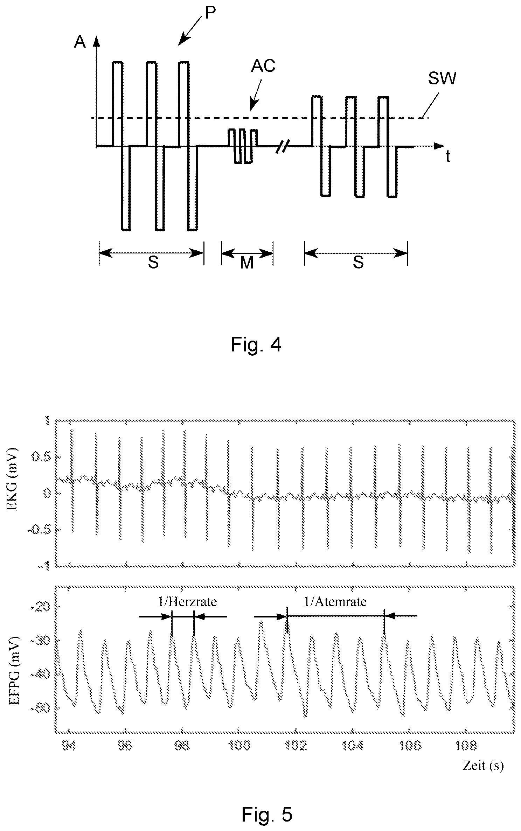

Fig. 4 zeigt den Verlauf eines Stroms mit der Stromstärke oder Spannung A über die Zeit t. In einer ersten Stimulationsphase S erzeugt der Stromgenerator 1 eine Folge von Stimulationsstromimpulsen P, die als Rechteckschwingung ausgebildet sind und über dem Schwellwert SW liegen, dessen Überschreitung einen Stimulationsreiz auslöst. In einer nachfolgenden Messphase M wird ein unterschwelliger Wechselstrom AC erzeugt, welcher der Gewebeimpedanzmessung dient. Danach folgt wieder eine Phase S mit einer Folge von Stimulationsstromimpulsen P.

Fig. 5 zeigt eine Beispielaufnahme eines Verlaufs der lokalen Gewebeimpedanz im Vergleich zu einem synchron aufgenommen Elektrokardiogramm (EKG). Das Impedanzkardiogramm wurde über drei im Ohr angebrachte Stimulationselektroden abgeleitet, wie in Fig. 3 dargestellt. Es sind die kardiale wie auch die respiratorische Aktivität deutlich erkennbar und können somit zur sensorlosen Regelung der aurikulären Stimulation herangezogen werden.

## Patentansprüche

1. Vorrichtung zur aurikulären Punktualstimulation eines Patienten, umfassend einen Stromgenerator (1) zur Erzeugung von Stimulationsstromimpulsen und elektrische Leitungen (2,3,4) zur Verbindung mit je einer am Ohr (12) zu positionierenden Elektrode (5,6,7), von denen wenigstens zwei mit den Stimulationsstromimpulsen beaufschlagbar sind, und weiters umfassend Messmittel zur Ermittlung wenigstens eines physiologischen Messwerts des Patienten, wobei die Messmittel zur Ermittlung des wenigstens einen physiologischen Messwerts mittels Impedanz-Plethysmographie über die Elektroden (5,6,7) ausgebildet sind, wobei der Stromgenerator (1) zur Erzeugung eines Wechselstroms ausgebildet ist, der über zwei der elektrischen Leitungen (2,4) und die zugehörigen Elektroden (5,7) in das Ohr (12) einleitbar ist, und wobei die Messmittel eine Messschaltung (8) zur Messung einer zwischen zwei Elektroden (6,7) abgegriffenen Gewebeimpedanz aufweisen, **dadurch gekennzeichnet, dass** der Stromgenerator (1) zur Erzeugung mehrerer Sequenzen von Stimulationsstromimpulsen mit Pausen zwischen den Sequenzen und zur Erzeugung des Wechselstroms in zumindest einer der Pausen ausgebildet ist.

2. Vorrichtung nach Anspruch 1, **dadurch gekennzeichnet, dass** die Messmittel eine mit der Messschaltung (8) verbundene Signalverarbeitungsschaltung (9) aufweisen, die ausgebildet ist, um aus dem zeitlichen Verlauf der Gewebeimpedanz den wenigstens einen physiologischen Messwert, wie z.B. die Herzrate, die Herzratenvariabilität, die Durchblutung, die Gefäßsteifigkeit und/oder die Atemfrequenz, zu ermitteln.

3. Vorrichtung nach Anspruch 1 oder 2, **dadurch gekennzeichnet, dass** der Stromgenerator (1) zur Erzeugung des Wechselstroms mit einer Wechselstromfrequenz von 5-100kHz ausgebildet ist.

4. Vorrichtung nach einem der Ansprüche 1 bis 3, **dadurch gekennzeichnet, dass** der Stromgenerator (1) zur Erzeugung der Stimulationsstromimpulse mit einer Pulsfrequenz von < 1kHz ausgebildet ist.

5. Vorrichtung nach einem der Ansprüche 1 bis 4, **dadurch gekennzeichnet, dass** der Stromgenerator (1) zur Erzeugung des Wechselstroms mit einer Stromamplitude von < 2mA ausgebildet ist.

6. Vorrichtung nach einem der Ansprüche 1 bis 5, **dadurch gekennzeichnet, dass** der Stromgenerator (1) zur Erzeugung der Stimulationsstromimpulse mit einer Stromamplitude von > 5mA ausgebildet ist.

7. Vorrichtung nach einem der Ansprüche 1 bis 6, **dadurch gekennzeichnet, dass** der Stromgenerator (1) zur Erzeugung der Stimulationsstromimpulse mit einer Stromamplitude ausgebildet ist, die mindestens dem 2-fachen, vorzugsweise mindestens dem 3-fachen, der Stromamplitude des Wechselstroms beträgt.

8. Vorrichtung nach einem der Ansprüche 1 bis 7 **dadurch gekennzeichnet, dass** eine Steuerschaltung (10) vorgesehen ist, die mit dem Stromgenerator (1) zur Änderung wenigstens eines Stimulationsstromparameters, wie z.B. der Pulsfrequenz, der Länge eines Bursts von Stimulationspulsen, der Stromamplitude und/oder des Tastverhältnisses der Stimulationsstromimpulse, zusammenwirkt.

9. Vorrichtung nach Anspruch 8, **dadurch gekennzeichnet, dass** die Messmittel mit der Steuerschaltung (10) zusammenwirken, um den wenigstens einen Stimulationsstromparameter in Abhängigkeit von dem wenigstens einen physiologischen Messwert einzustellen.

10. Vorrichtung nach einem der Ansprüche 1 bis 9, **dadurch gekennzeichnet, dass** der Stromgenerator (1) zur Variation der Frequenz des Wechselstroms ausgebildet ist, um eine dispersionsabhängige Charakterisierung der Gewebeimpedanz zu ermöglichen.

11. Vorrichtung nach einem der Ansprüche 1 bis 10, **dadurch gekennzeichnet, dass** die Vorrichtung eine Kommunikationsschnittstelle aufweist, welcher der wenigstens eine physiologische Messwert oder dessen zeitliche Änderung zugeführt ist und die zur Übermittlung des wenigstens einen physiologischen Messwerts oder von dessen zeitlicher Änderung an ein externe Empfangseinrichtung ausgebildet ist.

12. Vorrichtung nach einem der Ansprüche 1 bis 11, **dadurch gekennzeichnet, dass** die Kommunikationsschnittstelle zum Empfangen von Steuerbefehlen ausgebildet ist, die der Steuerschaltung zuführbar sind, um die Stimulationsstromparameter in Abhängigkeit von den Steuerbefehlen einzustellen.

## Claims

1. A device for auricular punctual stimulation of a patient, comprising an electrical current generator (1) for generating electrical stimulation pulses and electrical lines (2, 3, 4) for connecting to one electrode each (5, 6, 7) to be positioned on the ear (12), at least two of which electrodes can be acted on by the electrical stimulation pulses, and further comprising measurement means for determining at least one physiological measured value of the patient, wherein the measurement means are configured to determine the at least one physiological measured value by means of impedance plethysmography via the electrodes (5, 6, 7), wherein the electrical current generator (1) is configured to generate an alternating current, which can be introduced into the ear (12) via two of the electrical lines (2, 4) and the associated electrodes (5, 7), and wherein the measurement means have a measuring circuit (8) for measuring a tissue impedance tapped between two electrodes (6, 7), **characterised in that** the current generator (1) is configured to generate a plurality of sequences of electrical stimulation pulses with pauses between the sequences and to generate the alternating current in at least one of the pauses.

2. The device according to claim 1, **characterised in that** the measurement means include a signal processing circuit (9) which is connected to the measuring circuit (8) and which is configured to determine the at least one physiological measured value, such as the heart rate, the heart rate variability, the blood flow, the vascular stiffness and/or the respiratory rate, from the time course of the tissue impedance.

3. The device according to claim 1 or 2, **characterised in that** the electrical current generator (1) is configured to generate the alternating current with an alternating current frequency of 5-100kHz.

4. The device according to any one of claims 1 to 3, **characterised in that** the electrical current generator (1) is configured to generate the electrical stimulation pulses with a pulse frequency of < 1 kHz.

5. The device according to any one of claims 1 to 4, **characterised in that** the electrical current generator (1) is configured to generate the alternating current with a current amplitude of < 2 mA.

6. The device according to any one of claims 1 to 5, **characterised in that** the electrical current generator (1) is configured to generate the electrical stimulation pulses with an current amplitude of > 5mA.

7. The device according to any one of claims 1 to 6, **characterised in that** the electrical current generator (1) is configured to generate the electrical stimulation pulses with a current amplitude that is at least 2 times, preferably at least 3 times, the current amplitude of the alternating current.

8. The device according to any one of claims 1 to 7, **characterised in that** a control circuit (10) is provided, which interacts with the electrical current generator (1) to change at least one electrical stimulation parameter, such as the pulse frequency, the length of a burst of stimulation pulses, the current amplitude and/or the duty cycle of the electrical stimulation pulses.

9. The device according to claim 8, **characterised in that** the measurement means interact with the control circuit (10) to adjust the at least one electrical stimulation parameter as a function of the at least one physiological measured value.

10. The device according to any one of claims 1 to 9, **characterised in that** the electrical current generator (1) is configured to vary the frequency of the alternating current to allow a dispersion depending characterisation of the tissue impedance.

11. The device according to any one of claims 1 to 10, **characterised in that** the device includes a communication interface to which the at least one physiological measured value or its temporal change is supplied and which is configured to transmit the at least one physiological measured value or its temporal change to an external receiving device.

12. The device according to any one of claims 1 to 11, **characterised in that** the communication interface is configured to receive control commands that can be supplied to the control circuit in order to adjust the electrical stimulation parameters as a function of the control commands.

## Revendications

1. Dispositif pour la stimulation ponctuelle auriculaire d'un patient, comprenant un générateur de courant (1) pour produire des impulsions de courant de stimulation et des conducteurs électriques (2,3,4) pour la connexion avec respectivement une électrode (5,6,7) à positionner sur l'oreille (12), dont au moins deux peuvent être soumises aux impulsions de courant de stimulation, et comprenant de plus des moyens de mesure pour déterminer au moins une valeur physiologique mesurée du patient, les moyens de mesure pour déterminer ladite valeur physiologique étant conçus au moyen de la pléthysmographie d'impédance via les électrodes (5,6,7), le générateur de courant (1) étant conçu pour produire un courant alternatif qui peut être introduit dans l'oreille (12) via deux des conducteurs électriques (2,4) et les électrodes associées (5,7), et les moyens de mesure comprenant un circuit de mesure (8) pour mesurer une impédance tissulaire prélevée entre deux électrodes (6,7), **caractérisé en ce que** le générateur de courant (1) est conçu pour produire plusieurs séquences d'impulsions de courant de stimulation avec des pauses entre les séquences et pour produire le courant alternatif dans au moins une des pauses.

2. Dispositif selon la revendication 1, **caractérisé en ce que** les moyens de mesure comprennent un circuit de traitement de signal (9) connecté au circuit de mesure (8), qui est conçu pour déterminer, à partir de l'évolution temporelle de l'impédance tissulaire, l'au moins une valeur physiologique mesurée, telle que la fréquence cardiaque, la variabilité de la fréquence cardiaque, la circulation sanguine, la rigidité vasculaire et/ou la fréquence respiratoire.

3. Dispositif selon la revendication 1 ou 2, **caractérisé en ce que** le générateur de courant (1) est conçu pour produire le courant alternatif avec une fréquence de courant alternatif de 5-100 kHz.

4. Dispositif selon l'une des revendications 1 à 3, **caractérisé en ce que** le générateur de courant (1) est conçu pour produire les impulsions de courant de stimulation avec une fréquence d'impulsion de < 1kHz.

5. Dispositif selon l'une des revendications 1 à 4, **caractérisé en ce que** le générateur de courant (1) est conçu pour produire le courant alternatif avec une amplitude de courant de < 2mA.

6. Dispositif selon l'une des revendications 1 à 5, **caractérisé en ce que** le générateur de courant (1) est conçu pour produire les impulsions de courant de stimulation avec une amplitude de courant de > 5mA.

7. Dispositif selon l'une des revendications 1 à 6, **caractérisé en ce que** le générateur de courant (1) est conçu pour produire les impulsions de courant de stimulation avec une amplitude de courant qui est au moins 2 fois, de préférence au moins 3 fois, l'amplitude du courant alternatif.

8. Dispositif selon l'une des revendications 1 à 7, **caractérisé en ce qu'**un circuit de commande (10) est prévu, qui interagit avec le générateur de courant (1) pour modifier au moins un paramètre de courant de stimulation, tel que la fréquence d'impulsion, la longueur d'une rafale d'impulsions de stimulation, l'amplitude du courant et/ou le rapport cyclique des impulsions de courant de stimulation.

9. Dispositif selon la revendication 8, **caractérisé en ce que** les moyens de mesure interagissent avec le circuit de commande (10) pour régler l'au moins un paramètre de courant de stimulation en fonction de l'au moins une valeur physiologique mesurée.

10. Dispositif selon l'une des revendications 1 à 9, **caractérisé en ce que** le générateur de courant (1) est conçu pour faire varier la fréquence du courant alternatif afin de permettre une caractérisation dépendante de la dispersion de l'impédance tissulaire.

11. Dispositif selon l'une des revendications 1 à 10, **caractérisé en ce que** le dispositif comprend une interface de communication à laquelle l'au moins une valeur physiologique mesurée ou sa variation temporelle est fournie et qui est conçue pour transmettre l'au moins une valeur physiologique mesurée ou sa variation temporelle à un dispositif de réception externe.

12. Dispositif selon l'une des revendications 1 à 11, **caractérisé en ce que** l'interface de communication est conçue pour recevoir des commandes de contrôle, qui peuvent être fournies au circuit de commande, pour régler les paramètres de courant de stimulation en fonction des commandes de contrôle.
